(19) Europäisches Patentamt
European Patent Office
Office européen des brevets

(11) EP 2 773 314 B1

(12) **EUROPEAN PATENT SPECIFICATION**

(45) Date of publication and mention of the grant of the patent:
**06.12.2017 Bulletin 2017/49**

(21) Application number: **12775014.9**

(22) Date of filing: **16.10.2012**

(51) Int Cl.:
*A61K 8/06* (2006.01) *A61K 8/25* (2006.01)
*A61K 8/29* (2006.01) *A61K 8/898* (2006.01)
*A61Q 19/08* (2006.01)

(86) International application number:
**PCT/EP2012/070491**

(87) International publication number:
**WO 2013/064367 (10.05.2013 Gazette 2013/19)**

(54) **COSMETIC COMPOSITION**

KOSMETISCHE ZUSAMMENSETZUNG

COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.11.2011 PCT/CN2011/081826**

(43) Date of publication of application:
**10.09.2014 Bulletin 2014/37**

(73) Proprietors:
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**
- **Unilever PLC**
  **London, Greater London EC4Y 0DY (GB)**
  Designated Contracting States:
  **CY GB IE MT**

(72) Inventors:
- **KHOSHDEL, Ezat**
  **Wirral**
  **Bebington**
  **Merseyside CH63 3JW (GB)**
- **YUAN, Su**
  **Shanghai 200335 (CN)**
- **ZHANG, Qiqing**
  **Shanghai 200335 (CN)**

(74) Representative: **Corsten, Michael Allan**
**Unilever N.V.**
**Unilever Patent Group**
**Olivier van Noortlaan 120**
**3133 AT Vlaardingen (NL)**

(56) References cited:
**EP-A1- 2 280 040**
**WO-A1-2007/058380**
**WO-A1-2009/082565**
**FR-A1- 2 860 435**
**JP-A- 2008 143 820**
**US-A1- 2008 152 681**
**US-B1- 6 268 454**

- **STÉPHANIE POSTIAUX ET AL: "Soft focus of silica silylate aerogel", RESEARCH DISCLOSURE, MASON PUBLICATIONS, HAMPSHIRE, GB, vol. 516, no. 5, 1 April 2007 (2007-04-01) , page 347, XP007137202, ISSN: 0374-4353**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 773 314 B1

## Description

### Field of the invention

The present invention relates to cosmetic compositions, particularly cosmetic compositions suitable for reversible modulation of the optical appearance of skin.

### Background of the invention

Many consumers are concerned with the characteristics of their skin. For example, consumers with age spots or freckles often wish for such pigmented spots to be less pronounced. Other consumers may wish to reduce skin darkening caused by exposure to sunlight or to lighten or modulate their natural skin color.

To meet the needs of consumers products have been developed that influence skin chemistry like for example products that reduce pigment production in melanocytes (i.e. reduce melanogenesis). Such products are based on ingredients that alter the color of the skin itself and often tend to have low efficacy or undesirable side effects, such as for example toxicity or skin irritation.

Some consumers prefer to use products that alter the optical appearance of their skin in a reversible (non-permanent) way, and that do not (permanently) alter for example the color of the skin itself or interact with the chemistry of the skin. Products have been developed that contain particles, such as for example titanium dioxide, with a high refractive index that provide an optical whitening effect of the skin when these particles are applied on the skin e.g. as part of e.g. a skin cream. Such particles thus temporarily modulate the optical appearance of the skin they are applied on.

Wrinkles and fine lines in human skin are perceptible primarily as dark, non-reflective areas because of the way light falls and remains there. In contrast if light is reflected and diffused, the wrinkle appears less visible. Methods have been developed to throw light into the wrinkles and immediately eliminate the dark that makes the crease so obvious. Materials are used that generate an optical blurring but is still transparent enough to avoid a mask-like appearance. This is called the 'soft focus effect' and described by Dr. Emmert in "Quantification of the Soft-Focus Effect", Cosmetics and Toiletries, Vol. 111, 57-61 (1996).

GB2431103 (Oxonica Ltd.) describes the use as a skin toning agent and/or as a wrinkle-masking agent in a cosmetic formulation of a transition and/or lanthanide metal oxide (particularly one or more of TiO2, ZnO and CeO2) doped with one or more ions (particularly one of more of $Mn^{2+}$, $V^{5+}$ and $Cr^{3+}$) that impart optical absorption characteristics to the oxide.

Consumers prefer products that give a predictable modulation of the optical appearance of their skin. Not only concerning the effect immediately after applying cosmetic compositions, but also how the optical appearance of their skin develops over time. Usually the optical effect of cosmetic compositions is more pronounced directly after application with the optical effect diminishing over time.

JP 2008-143820 (KAO) describes cosmetic compositions comprising hydrophobic powder, co-polymer, nonionic water-soluble polymer and an oily component liquid at 25 degrees Celsius. The compositions are said to be stable over time, do not give a powdery feeling and have an excellent sense of use.

Consumers prefer products that are easy to use (like for example easy to apply to skin) and/or deliver excellent sensory benefits after topical application to skin (like for example non-stickiness or a silky sensation).

There is thus a need for cosmetic compositions that in a non-permanent way modulate the optical appearance of skin in a predictable way, especially over time after application on skin. Also, there is a need for cosmetic compositions that deliver such optical appearance benefits with good sensory properties like for example non-stickiness.

The inventors have developed cosmetic compositions that do not suffer from the aforementioned drawbacks.

EP 2 280 040 relates to an organopolysiloxane that exhibits good dispersion stability within not only organic oil agents but also cosmetic materials that contain a powder, and is able to produce a cosmetic material that exhibits excellent skin affinity, an organopolysiloxane that does not impart a sticky feeling, and exhibits excellent skin affinity and skin adhesion, and a cosmetic material including such an organopolysiloxane.

US 2008/152681 relates to A method of instantly reducing the appearance of wrinkles and skin imperfections while smoothing the skin, which comprises applying a cosmetic composition comprising a fractal particle based gel.

US 6 268 454 relates to a novel amino acid silicon polymer with good adhesion to the keratin layer of the skin, a method of preparing the amino acid silicon polymer, a cosmetic particles surface-treated with the amino acid silicon polymer, and a color cosmetic composition including the cosmetic particles.

### Summary of the invention

Accordingly the present invention relates to a cosmetic composition according to claim 1. The invention further relates to a method of modulating the optical appearance of skin comprising the step of applying to the skin the cosmetic

composition according to claim 1-14.

**Detailed description of the invention**

Weight percentage (wt%) is based on total weight of composition unless otherwise stated.

The cosmetic composition of the invention is a water-in-oil emulsion. Preferably the level of water is from 30 to 80 wt% and still even more preferably 40 to 70 wt%.

Optical appearance

The inventors have surprisingly found that the optical effect of optical light modifying agents when used in a cosmetic composition further comprising hydrophobically modified particles, amino functionalized silicone, water and viscosity modifying agent shows less decline over time after application on skin. Thus resulting in a more predictable modulation of the optical appearance of the skin over time.

The optical appearance of skin after application of cosmetic compositions of the invention can be measured by determining the opacity at 450 nm or the soft focus caused by the composition using the methods as described in the experimental section of this description.

The change over time of the optical appearance is defined as the opacity or soft focus after a determined time (t) divided by the opacity or soft focus directly after application (t=0) according to the method described in the experimental section of this description multiplied by 100%. For the purpose of this invention the resulting percentage is defined to be the optical appearance durability parameter 'OAD'.

For example, if for a composition the opacity at 450 nm is 0.124 directly after application and 0.117 after 30 minutes, the 'OAD after 30 minutes' is 94%.

For example, if for a composition the soft focus is 0.715 directly after application and 0.076 after 30 minutes, the 'OAD after 30 minutes' is 11%.

A composition according to any one of claims 1 to 13 having an optical appearance durability parameter OAD after 30 minutes of at least 50%, preferably at least 60%, more preferably at least 70%, even more preferably at least 80%, and still more preferably of at least 90%.

Preferably the optical appearance durability parameter OAD is based on opacity measurement.

Hydrophobically modified particles

The compositions of the invention comprise hydrophobically modified particles of silica, metal oxide and mixtures thereof. Preferably the hydrophobically modified particles comprise silica or titanium oxide, more preferably hydrophobically modified silica (silicone dioxide).

It is particularly preferred if the hydrophobically modified silica comprises the hydrophobic group of formula:

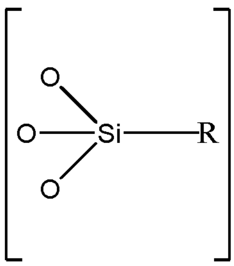

in which R is a $C_4$ to $C_{18}$ alkyl group.

Particularly preferred is the hydrophobically modified silicone dioxide comprising:

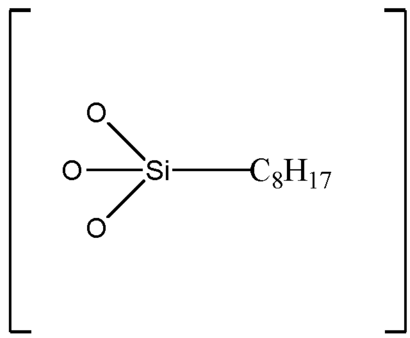

Preferred silicas are described in US 7 282 236 and made commercially available from suppliers like Evonik Degussa GmbH under the names Aerosil R812, R202 and R805. Silica having a C4 to C15 alkyl group attached to the silane are preferred. Particularly preferred is octylsilane comprising the group represented by the formula above (sold under the name Aerosil R805).

The hydrophobically modified silica preferably has a primary particle size (in its dry state) from 1 nm to 100 nm, more preferably from 5 nm to 70 nm.

Composition of the invention preferably comprise from 0.05 to 15 wt% of the total composition of hydrophobically modified silica, more preferably from 0.1 to 10 wt%, and most preferably from 0.2 to 5 wt%.

<u>Amino functionalized Silicone</u>

Compositions according to the invention comprise an amino functional silicone.

By "amino functionalized silicone" is meant a silicone containing at least one primary, secondary or tertiary amine group, or a quaternary ammonium group.

The primary, secondary, tertiary and/or quaternary amine groups may either form part of the main polymer chain or more preferably be carried by a side or pendant group carried by the polymeric backbone.

Suitable amino functionalized silicone polymers for use with the invention are described in US 4 185 087.

Amino functionalized silicones suitable for use in the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole %.

In a preferred embodiment, the functionalized polymers are of the amodimethicone having the general formula:

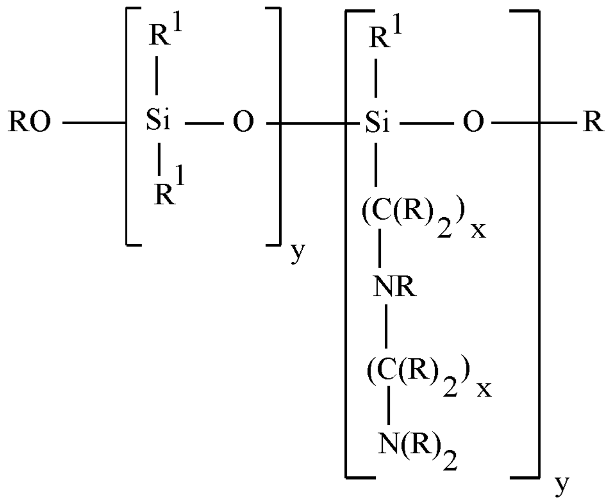

where each R is independently H, or a $C_{1-4}$ alkyl, preferably H;
each $R^1$ is independently OR or a $C_{1-4}$ alkyl; and
each x is independently an integer from 1 to 4 and each y is greater than zero and independently an integer to yield a polymer having a molecular weight from 500 to 1 million, and preferably from 750 to 25,000, and most preferably from 1,000 to 15,000.

Particularly preferred are silicones comprising an amino glycol copolymer. Especially preferred is Bis (C13-C15 alkoxy) PG amodimethicone such as DC 8500 by Dow Corning.

It is also within the scope of this invention for the functionalized polymer to comprise trimethylsilylamodimethicone.

Preferably the level of amino functionalized silicone polymers make up from 0.05 to 25 wt% of the total composition, more preferably from 0.05 to 15 wt%, even more preferably from 0.1 to 10%, and still even more preferably from 0.2 to 5 wt%.

Preferably the weight ratio of hydrophobically modified particle to amino functionalized silicone is from 3:1 to 1:50, more preferably from 1:1 to 1:10.

Viscosity modifying agents

Compositions of the of invention comprise a viscosity modifying agent selected from the group consisting of non-amino functionalized silicone, fatty acids, esters of fatty esters, hydrocarbons, fatty alcohols and mixtures thereof.

Suitable non-amino functionalized silicones include and preferably are polydiorganosiloxanes, in particular polydimethylsiloxanes which have the CTFA designation dimethicone. Also suitable for use in compositions of the invention are polydimethyl siloxanes having hydroxyl end groups, which have the CTFA designation dimethiconol. Also suitable for use in compositions of the invention are silicone gums having a slight degree of cross-linking, as are described for example in WO 96/31188. Suitable non-amino functionalized silicones are volatile silicone oils like for example cyclopentasiloxane (e.g. DC 245 Fluid from Dow Corning); non-volatile polydimethylsiloxane polymers like the DC 200 series of oils from Dow Corning with viscosity from 0.65 cst to 600,000 cst; silicone elastomer blends like DC 9041 (dimethicone and dimethicone crosspolymer from Dow Corning), DC 9045 and DC 9040 (cyclopentasiloxane and dimethicone crosspolymer from Dow Corning) DC 9546 (cyclopentasiloxane and dimethicone crosspolymer and dimethicone/vinyldimethicone crosspolymer and dimethiconol, from Dow Corning), DC 9506 (dimethicone/vinyldimethicone crosspolymer from Dow Corning); silicone elastomer suspensions like DC 9509 (dimethicone/vinyldimethicone crosspolymer and C12-14 Pareth-12 from Dow Corning).

Suitable fatty acids are fatty acid from 10 to 30 carbon atoms. Illustrative examples of such fatty acids include pelargonic, lauric, myristic, palmitic, stearic, isostearic, oleic, linoleic, arachidic, behenic or erucic acid, and mixtures thereof.

Esters of fatty acids include and preferably are alkyl ester of saturated fatty acids having 10-24 carbon atoms; ether-esters such as fatty acid esters of ethoxylated saturated fatty alcohols; polyhydric alcohol esters, ethylene glycol mono- and di-fatty acid esters, diethylene glycol mono- and di-fatty acid esters and so on; wax esters such as beeswax, spermaceti wax and tribehenin wax; sugar ester of fatty acids such as sucrose polybehenate and sucrose polycottonseedate; natural ester emollients based on mono- di- tri-glycerides.

Hydrocarbons include and preferably are petrolatum, mineral oil, C11-C13 isoparaffins, polytubenes, isohexadecane available commercially as permethyl 101 A from presperse Inc.

Fatty alcohols are aliphatic alcohols consisting of a chain of at least 8 carbon atoms. Preferably the fatty alcohol has a chain of 10 to 30 carbon atoms.

Preferably the viscosity modifying agent comprises at least a non-amino functionalized silicone and more preferably the viscosity modifying agent is selected from the group consisting of polydimethylsiloxane, pentasiloxane, dimethicone, mineral oil and mixtures thereof.

Preferred viscosity modifying agents are Dimethicone DC 200 series of oils from Dow Corning with viscosities from 0.65cst to 6000cst, mineral oil 70 SUS, glycerin, propylene glycol and dipropylene glycol.

Preferably the level of viscosity modifying agent is from 0.05 to 60 wt%, preferably 1 to 40 wt% and more preferably 5 to 30 wt%.

Preferably the weight ratio of viscosity modifying agent to amino functionalized silicone is from 10:1 to 1:50 and preferably from 3:1 to 1:20.

Optical light modifying agent

Compositions of the invention comprise an optical light modifying agent selected from the group consisting of particles with a refractive index from 1 to 3, agents providing a soft focus appearance and mixtures thereof.

Optical light modifying agents are a known ingredient in the field of cosmetic compositions. For example, particles with a high refractive index are used to provide for example a non-permanent whitening effect when applied to skin. Such particles can for example also be modified in such a way that they have color (i.e. not white). Preferably the particles have a refractive index from 1.5 to 3. Preferably the particle size is from 10 nm to 100 micrometer, more preferably 30 nm to 50 micrometer and even more preferably 50 nm to 1 micrometer.

Suitable particles include particles comprising titanium dioxide, zinc oxide, iron oxide, copper oxide, coral, cordierite, ivory, mother of pearl, jade, nephrite and jadeite.

Suitable particles further include polymers with a high refractive index such as Poly(pentabromophenyl methacrylate); Poly(pentabromophenyl acrylate); Poly(pentabromobenzyl methacrylate; Poly(pentabromobenzyl acrylate); Poly(2,4,6-tribromophenyl methacrylate); Poly(vinylphenylsulfide); Poly(1-napthyl methacrylate); Poly(2-vinylthiophene); Poly(2,6-dichlorostyrene); Poly(N-vinylphthalimide); Poly(2-chlorostyrene) and Poly(pentachlorophenyl methacrylate).

Particles providing a soft focus effect are well known in the field of cosmetic compositions and can suitably be obtained from commercial suppliers indicating so. Preferably such particles have a refractive index from 1 to 3 and more preferably from 1.3 to 2. Preferably these particles have a particle size from 50 nm to 100 micrometer, and preferably from 1 micrometer to 50 micrometers.

Suitable particles providing a soft focus effect include Nylon-6 powder (e.g. TR-1); Polymethyl Methcacrylate (e.g. GANZPEARL® GM-0600); Polyurethane microsphere (e.g. BPD-500W); Ethylene-Methyl Methacrylate copolymer (e.g. Flo-Beads SE-3107A); Mica and Titanium Dioxide and Ethylene/Methacrylate Copolymer and Isopropyl Titanium Triisostearate (e.g. SPC/KTZ Interval Blue-12); Nylon-12 and Chitosan (e.g. CT-2 Nylon SP-500); and Silica and C9-15 Fluoroalcohol Phosphate (e.g. PF-5 MSS-500/3N).

The optical light modifying agent may be made up of more than one material. It may for example be a particle composed of two or more different materials. The different materials may be just mixed (e.g. composites) or one material may be coated by another material. For example titanium dioxide particles may, at least partially, be coated with for example aluminum hydroxide.

Preferably the optical light modifying agent comprises titanium dioxide.

A preferred optical light modifying agent is SA-TR-10 from Miyoshi Kasei supplier (chemical name: Titanium dioxide, Aluminum hydroxide and dimethicone), having a particle size of 450 nm.

Preferably the level of optical light modifying agent is from 0.01 to 20 wt%, preferably 0.1 to 10 wt% and more preferably 0.1 to 5 wt%.

Further ingredients

In addition to water, organic solvents may be optionally included to act as carriers or to assist carriers within the compositions of the present invention. Illustrative and non-limiting examples of the types of organic solvents suitable for use in the present invention include alkanols like ethyl and isopropyl alcohol, mixtures thereof or the like.

In addition to the viscosity modifying agent in so far as these ingredients are not already present as an essential ingredient in compositions of the invention other additives may optionally be added.

Humectants of the polyhydric alcohol type may also be employed in the cosmetic compositions of this invention. The humectant often aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. The amount of humectant may range anywhere from 0.2 to 25 wt%, preferably from 0.3 to 20 wt% and more preferably from 0.5 to 15 wt%.

Thickeners may also be utilized as part of the cosmetic compositions of the invention. Typical thickeners include cross-linked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0 to 5 wt%, usually from 0.001 to 1 wt%, optimally from 0.01 to 0.5 wt%.

Collectively, the hydrophobically modified particles, amino functionalized silicone, water, viscosity modifying agent and the further ingredients being the co-solvents, silicones, esters, fatty acids, humectants and/or thickeners will constitute from 1 to 99.9 wt%, preferably from 80 to 99 wt% of the cosmetic composition.

Surfactants may also be present in cosmetic compositions of the present invention. Total concentration of the surfactant will range from 0 to 40 wt%, and preferably, from 0 to 20 wt%, optimally from 0 to 5 wt% of the composition. The surfactant may be selected from the group consisting of anionic, nonionic, cationic and amphoteric actives. Particularly preferred nonionic surfactants are those with a C10-C20 fatty alcohol or acid hydrophobe condensed with from 2 to 100 moles of ethylene oxide or propylene oxide per mole of hydrophobe; C2-C10 alkyl phenols condensed with from 2 to 20 moles of alkylene oxide; mono- and di- fatty acid esters of ethylene glycol; fatty acid monoglyceride; sorbitan, mono- and di- C8-C20 fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates,

alkylbenzene sulfonates, alkyl and dialkyl sulfosuccinates, C8-C20 acyl isothionates, acyl glutamates, C8-C20 alkyl ether phosphates and combinations thereof.

Perfumes may be used in the cosmetic composition of this invention. Illustrative non-limiting examples of the types of perfumes that may be used include those comprising terpenes and terpene derivatives like those described in Bauer, K., et al., Common Fragrance and Flavor Materials, VCH Publishers (1990).

Illustrative yet non-limiting examples of the types of fragrances that may be used in this invention include myrcene, dihydromyrenol, citral, tagetone, cis-geranic acid, citronellic acid, or cis-geranic acid nitrile, mixtures thereof or the like.

Preferably, the amount of fragrance employed in the cosmetic composition of this invention is in the range from 0 to 10 wt%, more preferably, 0.00001 to 5 wt%, most preferably, 0.0001 to 2 wt%.

Sunscreens include those materials commonly employed to block ultraviolet light. Illustrative compounds are the derivatives of PABA, cinnamate and salicylate. For example, avobenzophenone (Parsol 1789®) octyl methoxycinnamate and 2-hydroxy-4-methoxyl benzophenone (also known as oxybenzone) can be used. Octyl methoxycinnamate and 2-hydroxy-4-methoxy benzophenone are commercially available under the trademarks, Parsol MCX and Benzophenone-e, respectively. The exact amount of sunscreen employed in the compositions can vary depending upon the degree of protection desired from the sun's UV radiation.

In the event the cosmetic composition is an antiperspirant or deodorant composition, the same may comprise astringent actives. Examples include aluminum chlorohydrate, aluminum chlorhydrex, aluminum-zirconium chlorhydrex glycine, aluminum sulfate, zinc sulfate, zirconium and aluminum chlorohydroglycinate, zirconium hydroxychloride, zirconium and aluminum lactate, zinc phenolsulfonate and combinations thereof. Amounts of the astringents may range anywhere from 0.5 to 12 wt%.

Many cosmetic compositions, especially those containing water, should be protected against the growth of potentially harmful microorganisms. Anti-microbial compounds, such as triclosan, and preservatives are, therefore, typically necessary. Suitable preservatives include alkyl esters of p-hydroxybenzoic acid, hydantoin derivatives, propionate salts, and a variety of quaternary ammonium compounds. Particularly preferred preservatives of this invention are methyl paraben, propyl paraben, phenoxyethanol and benzyl alcohol. Preservatives will usually be employed in amounts ranging from 0.1 to 2 wt% of the composition.

Still other optional ingredients that may be used with the cosmetic composition of this invention include dioic acids (e.g., malonic acid, sebacic acid), antioxidants like vitamin E, vitamins, like niacinamide and vitamin C and its derivatives, recorcinols and its derivatives (including those esterified with, for example, ferulic acid, vanillic acid or the like) and retinoids, including retinoic acid, retinal, retinol and retinyl esters, conjugated linoleic acid, petroselinic acid and mixtures thereof, as well as any other conventional ingredients well known for wrinkle-reducing, skin whitening, anti-acne effects and reducing the impact of sebum.

<u>Cosmetic compositions</u>

Cosmetic composition, as used herein, is meant to include a composition for topical application to skin of mammals, especially humans. Compositions of the invention are typically 'leave-on' compositions, and is meant to include conditioners or tonics, lipsticks, color cosmetics, and general topical compositions that in some fashion and at the very least modulate the optical appearance of the surface applied to. Other benefits may include skin moisturizing, decreasing the effect of sebum on the skin and skin wrinkle reducing.

Cosmetic compositions of the invention can be in the form of a liquid, lotion, cream, serum, gel, or toner. The composition of this invention is one that at the very least is suitable to modulate the optical appearance of skin in a non-permanent way (i.e. the modulation of the optical appearance is reversed when the composition is removed like for example rinsed off) when skin is meant to include skin on the face, neck, chest, back, arms, hands, legs, scalp and underarm.

Preferably the viscosity of compositions of the invention at 25 degrees Celsius comprising hydrophobically modified particles, amino functionalized oil, water, viscosity modifying oil and further ingredients except the optical light modifying agent is from $10^3$ Pa.s to $10^6$ Pa.s, more preferably $2*10^3$ Pa.s to $5*10^5$ Pa.s.

The viscosity of the final cosmetic composition at 25 degrees Celsius preferably is from $10^3$ Pa.s to $10^5$ Pa.s, and more preferably $5*10^4$ Pa.s to $5*10^5$ Pa.s.

Often, the cosmetic composition of the present invention has a melting point from about 30 to 45 degrees Celsius. In an especially preferred embodiment, the cosmetic composition of the present invention has a pH from 4.5 to about 8 at 25 degrees Celsius. More preferably from 5 to 7.

<u>Method of modifying the optical appearance of skin</u>

The invention also concerns a method of modulating the optical appearance of skin comprising the step of

applying to the skin the cosmetic composition of the invention as described.

The method concerns at least the non-permanent modulation of the optical appearance of skin (i.e. the modulation of the optical appearance is reversed when the composition is removed like for example rinsed off). Preferably the method only concerns non-permanent modulation of the optical appearance of skin.

The non-permanent modulation of the optical appearance of skin typically concerns opacity and/or soft focus. Preferably the optical appearance concerns opacity.

The invention is now illustrated by the following non-limiting examples.

## Examples

Primary particle size

Primary particle sizes can be derived from Transmission Electron Microscopy according to the method described by S. Gu et al in Journal of Colloid and Interface Science 289 (2005) 419-426.

Viscosity measurement

Viscosity is a measure of the resistance of fluid to an applied stress. A MCR 501 rheometer (from Anton Paar Instrument Ltd) is used to measure the viscosities. The rheometer is connected to a compressed air source to maintain a pressure of 5 bar, to ensure a continuous rotation rate. A parallel plate was used having geometry of 25 mm in diameter. An automatic controlled rate mode is used to measure the forces in a shear rate range from 0.001 to 100 $s^{-1}$ at a temperature of 25 degrees Celsius.

Opacity measurement

Opacity was measured by a portable spectrophotometer (MINOLTA CM2600d in SCE mode) at a temperature of about 23 degrees Celsius and 45% relative humidity. A product film with a thickness of 75 micrometers was applied on a black and white card using a film applicator. The opacity (450nm) is defined as the ratio between the reflectance of the product film on the black and white cards at a wavelength of 450nm.

Soft focus measurement

The soft focus index was measured with a Goniometer after applying a product film of 75 micrometers on the glass slide using a SHEEN cubic film applicator at a temperature of about 23 degrees Celsius and 45% relative humidity.

The soft focus (SF) of the product film was calculated with:

$$SF = \int_{ST+9}^{ST+90} T(\theta)d\theta \Big/ \int_{ST}^{ST+90} T(\theta)d\theta$$

where T(θ) is the transmittance at angle θ; and ST is specular transmittance. The incident angle was set at 48°.

Formulation process

To form compositions of the invention the hydrophobically modified silica particles are dispersed in the silicone oils (amino functionalized and non-amino functionalized) (Part I). The ingredients of Part II are combined under stirring until a uniform phase forms. Part I and Part II are then mixed in a steel vessel. The ingredients of Part III are combined under stirring and the ingredients of Part IV (if any) are added. The mixture of Part III and Part IV is then combined with the mixture of Part I and Part II by light homogenization to form a uniform structure. Further ingredients (if any) are added. The process takes place under ambient temperature.

Ingredients used

Aerosil R805 ex. Evonik Degussa GmbH; DC 8500, DC 200 fluid 5cst, DC 200 fluid 12500 cst, DC 9045 all ex. Dow corning; SA-TR-10 ex. Miyoshi Kasei; Mineral oil ex. Sonneborn; Z cote HP-1 ex. BASF; CM3W40DRF ex. KOBO; GANZPEARL® GM-0600 ex. GANZ Chemical.

Comparatives A and B

Table 1, Composition of comparatives A and B (in wt%)

| Trade name | Chemical name | Comp. A | Com. B |
|---|---|---|---|
| Aerosil R805 | Hydrophobic silica | 4.9 | - |
| DC 8500 | Bis (C13-15 Alkoxy) PG Amodimethicone | 44.5 | 49.4 |
| H2O | Water | to 100 | to 100 |
| SA-TR-10 | Titanium dioxide (And) Aluminum hydroxide (And) Dimethicone | 1.2 | 1.2 |

Table 2, Opacity over time of comparatives A and B

| | Opacity (450nm) / Time | | | | | | |
|---|---|---|---|---|---|---|---|
| | 0 min | 5 min | 10 min | 15 min | 20 min | 25 min | 30 min |
| Comp. A | 0.306 | 0.249 | 0.227 | 0.195 | 0.191 | 0.179 | 0.190 |
| Comp. B | 0.254 | 0.166 | 0.128 | 0.126 | 0.125 | 0.126 | 0.131 |

Comparative examples A (no viscosity modifying agent) and B (no modified silica and no viscosity modifying agent) show a rapid decline in opacity over 30 minutes.

Examples 1 and 2

Table 3, Composition of examples 1 and 2 (in wt%)

| Trade name | Chemical name | Ex. 1 | Ex. 2 |
|---|---|---|---|
| Aerosil R805 | Hydrophobic silica | 4.9 | 9.9 |
| DC 8500 | Bis (C13-15 Alkoxy) PG Amodimethicone | 4.5 | 23.7 |
| DC 200 fluid 5cst | Polydimethylsiloxane | 40.0 | - |
| Mineral oil | Paraffinum Liquidum | - | 15.8 |
| H2O | Water | to 100 | to 100 |
| SA-TR-10 | Titanium dioxide (And) Aluminum hydroxide (And) Dimethicone | 1.2 | 1.2 |

Table 4, Opacity over time of examples 1 and 2

| | Opacity (450nm) / Time | | | | | | |
|---|---|---|---|---|---|---|---|
| | 0 min | 5 min | 10 min | 15 min | 20 min | 25 min | 30 min |
| Ex. 1 | 0.145 | 0.146 | 0.142 | 0.151 | 0.147 | 0.147 | 0.150 |
| Ex. 2 | 0.178 | 0.175 | 0.172 | 0.169 | 0.162 | 0.162 | 0.162 |

Examples 1 and 2 (containing different viscosity modifying agents) show little if any decline in opacity over 30 minutes.

Examples 3 and 4

Table 5, Composition of examples 3 and 4 (in wt%)

| Trade Name | Chemical name | Ex. 3 | Ex. 4 |
|---|---|---|---|
| Aerosil R805 | Hydrophobic silica | 4.9 | 4.9 |
| DC 8500 | Bis (C13-15 Alkoxy) PG Amodimethicone | 4.4 | 4.4 |
| DC 200 fluid 5cst | Polydimethylsiloxane | 39.7 | 39.7 |
| H2O | Water | to 100 | to 100 |
| Z cote HP-1 | Zinc Oxid (And) Triethoxycaprylysilane | 2.0 | - |
| CM3W40DRF | Cyclomethicone (And) Rec Iron Oxide (And) C9-15 Fluoroalcohol Phosphates (And) Polyglyceryl-4 Isostearate (And) Cetyl Dimethicone Copolyol (And) Hexyl Laurate | - | 2.0 |

Table 6, Opacity over time of examples 3 and 4

| | Opacity (450nm) / Time | | | | | | |
|---|---|---|---|---|---|---|---|
| | 0 min | 5 min | 10 min | 15 min | 20 min | 25 min | 30 min |
| Ex. 3 | 0.065 | 0.071 | 0.071 | 0.071 | 0.072 | 0.073 | 0.071 |
| Ex. 4 | 0.417 | 0.442 | 0.43 | 0.419 | 0.416 | 0.414 | 0.413 |

Examples 3 and 4 (containing different optical light modifying agents) show little if any decline in opacity over 30 minutes.

Example 5 and comparatives C and D

Table 7, Composition of example 5 (in wt%)

| Part | Trade name | Chemical name | Ex. 5 |
|---|---|---|---|
| I | Aerosil R805 | Hydrophobic silica | 1.00 |
| | DC 8500 | Bis (C13-15 Alkoxy) PG Amodimethicone | 0.90 |
| | DC 200 fluid 5cst | Polydimethylsiloxane | 8.10 |
| | glycerine | Glycerin | 20.00 |
| II | Parsol MCX | Ethylhexyl Methoxycinnamate | 6.00 |
| | GANZPEARL® GM-0600 | Polymethyl Methacrylate | 4.00 |

(continued)

| Part | Trade name | Chemical name | Ex. 5 |
|---|---|---|---|
| III | H2O | Water | to 100 |
| | EDTA.2Na | EDTA Disodium | 0.05 |
| | phenoxyethanol | Phenoxyethanol | 0.40 |
| | U21 | Acrylates/C10-30 alkyl acrylate crosspolymer | 0.30 |
| | NaOH (20% solution) | Sodium hydroxide | 0.30 |
| | glycerin | Glycerin | 5.00 |
| | methylparaben | Methylparaben | 0.20 |
| | propylparaben | Propylparaben | 0.10 |
| IV | DC 200 fluid 5cst | Polydimethylsiloxane | 6.00 |

Table 8, Composition of comparative C (in wt%)

| Part | Trade name | Chemical name | Comp. C |
|---|---|---|---|
| I | H2O | Water | to 100 |
| | EDTA.2Na | EDTA Disodium | 0.05 |
| | Methylparaben | Methylparaben | 0.20 |
| | Emulgin SG | E, Sucrose Polystearate and. Hydrogenated Polyisobutane | 0.50 |
| II | Cosmedia SP | T, Acrylic acid homopolymer, sodium salt | 0.35 |
| | Butylene glycol | H, Butylene glycol | 1.00 |
| | Glycerin | Glycerin | 3.00 |
| | H2O | Water | 2.00 |
| III | Propylparaben | Propylparaben | 0.10 |
| | PCL solid | O, Stearyl Heptanoate, Stearyl Caprylate | 1.00 |
| | Cetearyl alcohol | C16-18, O | 2.40 |
| | Cetoil CC | Dicaprylyl Carbonate | 4.00 |
| | Puresyn 6 | O, Hydrogenated Polydecene | 3.00 |
| IV | Emulgade SUCRO | E, Sodium Stearoyl Gluamate | 1.00 |
| | DC 200 fluid 200cst | Polydimethylsiloxane | 2.00 |
| V | Parsol MCX | Ethylhexyl Methoxycinnamate | 6.00 |
| VI | GANZPEARL® GM-0600 | Polymethyl Methacrylate | 4.00 |
| VII | GTCC | E, Caprylic / Capric Triglyceride | 2.00 |
| VIII | Covacryl MV 60 | Sodium Polyacrylate, T | 0.20 |
| | Butylene glycol | H, Butylene glycol | 1.00 |
| | H2O | Water | 2.00 |
| IX | H2O | Water | 15.25 |

Table 9, Composition of comparative D (in wt%)

| Part | Trade name | Chemical name | Comp. D |
|---|---|---|---|
| I | Parsol MCX | Ethylhexyl Methoxycinnamate | 2.00 |
| | DC 9045 | Cyclopentasiloxane (and) Dimethicone Crosspolymer | 26.50 |
| II | KF6017 | PEG-10 Dimethicone | 1.19 |
| | GTCC | E, Caprylic / Capric Triglyceride | 2.50 |
| | silsoft 034 | Caprylyl methicone | 0.50 |
| | Parsol MCX | Ethylhexyl Methoxycinnamate | 4.00 |
| | DC245 | Cyclopentasiloxane | 0.50 |
| | GANZPEARL® | Polymethyl Methacrylate | 4.00 |
| | GM-0600 | | |
| | Bentone 38VCG | Disteardimonium Hectorite | 0.27 |
| III | H2O | Water | to 100 |
| | Glycerine | Glycerine | 7.25 |

Table 10, Soft focus over time of example 5 and comparatives C and D

| | Soft Focus / Time | | | | | | |
|---|---|---|---|---|---|---|---|
| | 0 min | 5 min | 10 min | 15 min | 20 min | 25 min | 30 min |
| Ex. 5 | 0.175 | 0.091 | 0.081 | 0.144 | 0.179 | 0.191 | 0.191 |
| Comp. C | 0.715 | 0.676 | 0.570 | 0.256 | 0.113 | 0.081 | 0.076 |
| Comp. D | 0.582 | 0.361 | 0.080 | 0.054 | 0.059 | 0.059 | 0.062 |

Example 5 and comparative examples C and D (all containing the optical light modifying agent Polymethyl Methacrylate, but in a different matrix) show that in a composition according to the invention there is little if any decline in soft focus.

Examples 6 to 9

Table 11, Composition of examples 6 to 9 (in wt%)

| Part | Trade name | Chemical name | Ex. 6 | Ex. 7 | Ex. 8 | Ex. 9 |
|---|---|---|---|---|---|---|
| I | Aerosil R805 | Hydrophobic silica | 1.00 | 1.00 | 0.83 | 0.91 |
| | DC 8500 | Bis (C13-15 Alkoxy) PG Amodimethicone | 0.90 | 4.50 | 3.75 | 2.18 |
| | DC 200 fluid 5cst | Polydimethylsiloxane | 8.10 | - | - | - |
| | DC 200 fluid 12500 cst | Polydimethylsiloxane | - | 4.50 | - | - |
| | DC 9045 | Cyclopentasiloxane and Dimethicone Crosspolymer | - | - | 3.75 | - |
| | Mineral oil | Mineral oil 70 SUS | - | - | - | 1.45 |
| | Glycerin | Glycerin | 20.00 | 20.00 | 8.33 | 18.18 |

(continued)

| Part | Trade name | Chemical name | Ex. 6 | Ex. 7 | Ex. 8 | Ex. 9 |
|---|---|---|---|---|---|---|
| II | Parsol MCX | Ethylhexyl Methoxycinnamate | 6.00 | 6.00 | 5.00 | 5.45 |
| | SA-TR-10 | Titanium dioxide (And) Aluminum hydroxide (And) Dimethicone | 0.60 | 0.60 | 0.50 | 0.6 |
| III | H2O | Water | to 100 | to 100 | to 100 | to 100 |
| | EDTA.2Na | EDTA Disodium | 0.05 | 0.05 | 0.04 | 0.04 |
| | Phenoxyethanol | Phenoxyethanol | 0.40 | 0.40 | 0.33 | 0.36 |
| | U21 | Acrylates/C10-30 alkyl acrylate crosspolymer | 0.30 | 0.30 | 0.25 | 0.27 |
| | NaOH (sol. 20%) | Sodium hydroxide | 0.30 | 0.30 | 0.25 | 0.27 |
| | Glycerin | Glycerin | 5.00 | 5.00 | 4.17 | 4.54 |
| | Methylparaben | Methylparaben | 0.20 | 0.20 | 0.17 | 0.18 |
| | Propylparaben | Propylparaben | 0.10 | 0.10 | 0.09 | 0.09 |
| IV | DC 200 fluid 1.5cst | Polydimethylsiloxane | 6.00 | 6.00 | 30.00 | 14.54 |

Table 12, Opacity over time of examples 6 to 9

| | Opacity (450nm) / Time | | | | | | |
|---|---|---|---|---|---|---|---|
| | 0 min | 5 min | 10 min | 15 min | 20 min | 25 min | 30 min |
| Ex. 6 | 0.086 | 0.073 | 0.069 | 0.068 | 0.068 | 0.066 | 0.068 |
| Ex. 7 | 0.124 | 0.112 | 0.116 | 0.117 | 0.117 | 0.113 | 0.117 |
| Ex. 8 | 0.089 | 0.083 | 0.083 | 0.087 | 0.086 | 0.091 | 0.088 |
| Ex. 9 | 0.082 | 0.080 | 0.082 | 0.082 | 0.081 | 0.082 | 0.081 |

Examples 6 to 9 (containing different viscosity modifying agents) show little if any decline in opacity over 30 minutes.

## Claims

**1.** A cosmetic composition comprising a water-in-oil emulsion comprising

a. hydrophobically modified particles selected from the group consisting of silica, metal oxide and mixtures thereof;
b. amino functionalized silicone; and
c. water;

the cosmetic composition further comprising a

d. viscosity modifying agent selected from the group consisting of non-amino functionalized silicone, fatty acids, esters of fatty esters, hydrocarbons, fatty alcohols and mixtures thereof;
e. optical light modifying agent selected from the group consisting of particles with a refractive index from 1 to

3, agents providing a soft focus appearance and mixtures thereof;

wherein the level of water is from 30 to 90 wt%, preferably from 30 to 80 wt%; and wherein the agents providing a soft focus appearance are selected from Nylon-6 powder; Polymethyl Methcacrylate; Polyurethane microsphere; Ethylene-Methyl Methacrylate copolymer; Mica and Titanium Dioxide and Ethylene/Methacrylate Copolymer and Isopropyl Titanium Triisostearate; Nylon-12 and Chitosan; and Silica and C9-15 Fluoroalcohol Phosphate.

**2.** A composition according to claim 1 wherein the hydrophobically modified particles comprise silica or titanium oxide, more preferably comprise silica.

**3.** A composition according to claim 1 or claim 2 wherein the hydrophobically modified silica comprise

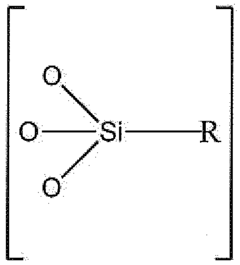

wherein R is a C4 to C18 alkyl group, more preferably a C8H17 alkyl group.

**4.** A composition according to any one of claims 1 to 3 wherein the hydrophobically modified particle has a primary particle size from 1 nm to 100 nm, preferably from 5 nm to 70 nm.

**5.** A composition according to any one of claims 1 to 4 wherein the amino functionalized silicone comprises an amino glycol copolymer.

**6.** A composition according to any one of claims 1 to 5 wherein the level of amino functionalized silicone is from 0.05 to 25 wt%, preferably 0.05 to 15 wt%, more preferably 0.1 to 10 wt% and even more preferably 0.2 to 5 wt% of the total composition.

**7.** A composition according to any one of claims 1 to 6 wherein the weight ratio of hydrophobically modified particle to amino functionalized silicone is from 3:1 to 1:50, preferably from 1:1 to 1:10.

**8.** A composition according to any one of claims 1 to 7 wherein the viscosity modifying agent comprises at least a non-amino functionalized silicone and preferably the viscosity modifying agent is selected from the group consisting of polydimethylsiloxane, pentasiloxane, dimethicone, mineral oil and mixtures thereof.

**9.** A composition according to any one of claims 1 to 8 wherein the level of viscosity modifying agent is from 0.05 to 60 wt%, preferably 1 to 40 wt% and more preferably 5 to 30 wt%.

**10.** A composition according to any one of claims 1 to 9 wherein the weight ratio of viscosity modifying agent to amino functionalized silicone is from 10:1 to 1:50 and preferably from 3:1 to 1:20.

**11.** A composition according to any one of claims 1 to 10 wherein the optical light modifying agent comprises titanium dioxide.

**12.** A composition according to any one of claims 1 to 11 wherein the level of optical light modifying agent is from 0.01 to 20 wt%, preferably 0.1 to 10 wt% and more preferably 0.1 to 5 wt%.

**13.** A composition according to any one of claims 1 to 12 wherein the viscosity of the composition at 25 degrees Celsius is from $10^3$ Pa.s to $10^5$ Pa.s, and preferably $5*10^4$ Pa.s to $5*10^5$ Pa.s.

**14.** A composition according to any one of claims 1 to 13 having an optical appearance durability parameter OAD after 30 minutes of at least 50%, preferably at least 60%, more preferably at least 70%, even more preferably at least

80%, and still more preferably of at least 90%; wherein the OAD is defined as the opacity or soft focus after a determined time (t) divided by the opacity or soft focus directly after application (t=0) multiplied by 100%; wherein opacity is measured by a portable spectrophotameter (MINOLTA CM2600d in SCE mode) at a temperature of about 23 degrees Celsius and 45% relative humidity with a product film with a thickness of 75 micrometers applied on a black and white card using a film applicator, with the opacity (450nm) being the ratio between the reflectance of the product film on the black and white cards at a wavelength of 450nm; and wherein the soft focus index is measured with a Goniometer after applying a product film of 75 micrometers on the glass slide using a SHEEN cubic film applicator at a temperature of about 23 degrees Celsius and 45% relative humidity, with the soft focus (SF) of the product film being calculated with:

$$SF = \int_{ST+9}^{ST+90} T(\theta)d\theta \Big/ \int_{ST}^{ST+90} T(\theta)d\theta$$

where T(θ) is the transmittance at angle θ; ST is specular transmittance; and the incident angle is set at 48°.

**15.** A method of modulating the optical appearance of skin comprising the step of applying to the skin a cosmetic composition comprising a water-in-oil emulsion according to any one of claims 1 to 14.

**Patentansprüche**

**1.** Kosmetische Zusammensetzung umfassend eine Wasser-in-Öl Emulsion umfassend

a. hydrophob modifizierte Partikel ausgewählt aus der Gruppe, bestehend aus Silica, Metalloxid und Mischungen davon;
b. aminofunktionalisiertes Silikon;
c. Wasser;

wobei die kosmetische Zusammensetzung ferner umfasst ein

d. ein viskositätsmodifizierendes Mittel ausgewählt aus der Gruppe, bestehend aus nichtaminofunktionalisiertem Silikon, Fettsäuren, Estern von Fettsäure, Kohlenwasserstoffen, Fettalkoholen und Mischungen davon;
e. ein optisches Licht modifizierendes Mittel ausgewählt aus der Gruppe, bestehend aus Partikeln mit einem Brechungsindex von 1 bis 3, Mitteln, die ein weichgezeichnetes Erscheinungsbild vermitteln und Mischungen davon;

wobei der Gehalt an Wasser von 30 bis 90 Gew.-%, vorzugsweise von 30 bis 80 Gew.-% beträgt und wobei die Mittel, die ein weichgezeichnetes Erscheinungsbild vermitteln, aus Nylon-6-Pulver; Polymethylmethacrylat; Polyurethanmikrosphären; Ethylen-methylmethacrylat-Copolymer, Glimmer und Titandioxid und Ethylen/Methacrylat-Copolymer und Isopropyltitantriisostearat, Nylon -12 und Chitosan, und Silica und C9-15 Fluoralkoholphosphat ausgewählt sind.

**2.** Zusammensetzung gemäß Anspruch 1, wobei die hydrophob modifizierten Partikel Silica oder Titanoxid, bevorzugt Silica, umfassen.

**3.** Zusammensetzung gemäß Anspruch 1 oder 2, wobei das hydrophob modifizierte Silica

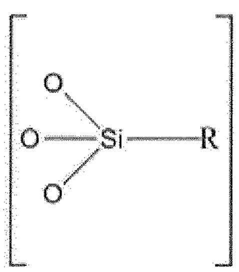

umfasst, wobei R eine C4-bis C18-Alkylgruppe, vorzugsweise eine C8H17-Alkylgruppe, darstellt.

**4.** Zusammensetzung gemäß irgendeinem der Ansprüche 1 bis 3, wobei das hydrophob modifizierte Partikel eine Primärpartikelgröße von 1 nm bis 100 nm, vorzugsweise von 5 nm bis 70 nm, aufweist.

**5.** Zusammensetzung gemäß irgendeinem der Ansprüche 1 bis 4, wobei das aminofunktionalisierte Silikon ein Aminglykol-Copolymer umfasst.

**6.** Zusammensetzung gemäß irgendeinem der Ansprüche 1 bis 5, wobei der Gehalt des aminofunktionalisierten Silikons von 0,05 bis 25 Gew.-%, vorzugsweise von 0,05 bis 15 Gew.-%, besonders bevorzugt von 0,1 bis 10 Gew.-% und ganz besonders bevorzugt von 0,2 bis 5 Gew.-%, bezogen auf die gesamte Zusammensetzung, beträgt.

**7.** Zusammensetzung gemäß irgendeinem der Ansprüche 1 bis 6, wobei das Gewichtsverhältnis des hydrophob modifizierten Partikels zu aminofunktionalisiertem Silikon von 3:1 bis 1:50, vorzugsweise von 1:1 bis 1:10, beträgt.

**8.** Zusammensetzung gemäß irgendeinem der Ansprüche 1 bis 7, wobei das viskositätsmodifizierende Mittel mindestens ein nicht-aminofunktionalisiertes Silikon umfasst und das nicht-aminofunktionalisierte Silikon vorzugsweise aus der Gruppe ausgewählt ist, bestehend aus Polydimethylsiloxan, Pentasiloxan, Dimethicone, Mineralöl und Mischungen davon.

**9.** Zusammensetzung gemäß irgendeinem der Ansprüche 1 bis 8, wobei der Gehalt an viskositätsmodifzierendem Mittel von 0,05 bis 60 Gew.-%, vorzugsweise von 1 bis 40 Gew.-% und besonders bevorzugt von 5 bis 30 Gew.-% beträgt.

**10.** Zusammensetzung gemäß irgendeinem der Ansprüche 1 bis 9, wobei das Gewichtsverhältnis von viskositätsmodifizierendem Mittel zu aminofunktionalisiertem Silikon von 10:1 bis 1:50 und vorzugsweise von 3:1 bis 1:20 beträgt.

**11.** Zusammensetzung gemäß irgendeinem der Ansprüche 1 bis 10, wobei das optisches Licht modifizierende Mittel Titandioxid umfasst.

**12.** Zusammensetzung gemäß irgendeinem der Ansprüche 1 bis 11, wobei der Gehalt an optisches Licht modifizierendem Mittel von 0,01 bis 20 Gew.-%, vorzugsweise von 0,1 bis 10 Gew.-% und besonders bevorzugt von 0,1 bis 5 Gew.-% beträgt.

**13.** Zusammensetzung gemäß irgendeinem der Ansprüche 1 bis 12, wobei die Viskosität der Zusammensetzung bei 25 °C von $10^3$ Pa.s bis $10^5$ Pa.s und vorzugsweise von $5*10^4$ Pa.s bis $5*10^5$ Pa.s beträgt.

**14.** Zusammensetzung gemäß irgendeinem der Ansprüche 1 bis 13, die einen Beständigkeitsparameter für das optische Erscheinungsbild (OAD) nach 30 Minuten von mindestens 50 %, vorzugsweise mindestens 60 % und besonders bevorzugt von mindestens 70 %, ganz besonders bevorzugt von mindestens 80 % und noch bevorzugter von mindestens 90 % aufweist, wobei der OAD als die Opazität oder die Weichzeichnung nach einer bestimmten Zeit (t), dividiert durch die Opazität oder die Weichzeichnung direkt nach dem Auftragen (t=0) multipliziert mit 100 %, definiert wird, wobei die Opazität mit einem tragbaren Spektrophotometer (MINOLTA CM2600d im SCE Modus) bei einer Temperatur von etwa 23°C und 45% relativer Feuchtigkeit mit einem Produktfilm einer Dicke von 75 Mikrometern, der auf eine Schwarz-Weiß-Karte unter Verwendung eines Applikators aufgetragen wird, gemessen wird, wobei die Opazität (450 nm) das Verhältnis zwischen dem Reflexionsgrad des Produktfilms auf den Schwarz-Weiß-Karten bei einer Wellenlänge von 450 nm ist; und wobei der Weichzeichnungsindex mit einem Goniometer nach Auftragen eines Produktfilms von 75 Mikrometern auf den Glasobjektträger unter Verwendung eines kubischen Filmapplikators SHEEN bei einer Temperatur von etwa 23°C und 45% relativer Feuchtigkeit gemessen wird, wobei die Weichzeichnung (SF) des Produkts durch

$$SF = \int_{ST+9}^{ST+90} T(\theta)d\theta / \int_{ST}^{ST+90} T(\theta)d\theta$$

berechnet wird, wobei T(θ) die Transmission bei einem Winkel θ ist, ST die gerichtete Transmission ist; und der Auftreffwinkel auf 48°C eingestellt wird.

**15.** Verfahren zur Modulierung der optischen Erscheinung der Haut umfassend den Schritt des Auftragens einer kosmetischen Zusammensetzung, umfassend eine Wasser-in-Öl Emulsion gemäß irgendeinem der Ansprüche 1 bis 14, auf die Haut.

**Revendications**

**1.** Composition cosmétique comprenant une émulsion eau-dans-huile comprenant

a. des particules hydrophobiquement modifiées choisies dans le groupe constitué de silice, d'oxyde de métal et de mélanges de ceux-ci ;
b. un silicone amino fonctionnalisé ; et
c. de l'eau ;

la composition cosmétique comprenant de plus un

d. agent de modification de la viscosité choisi dans le groupe constitué de silicone non-amino fonctionnalisé, d'acides gras, d'esters d'esters gras, d'hydrocarbures, d'alcools gras et de mélanges de ceux-ci ;
e. agent de modification de lumière optique choisi dans le groupe constitué de particules avec un indice de réfraction de 1 à 3, d'agents fournissant une apparence floutée et de mélanges de ceux-ci ;

dans laquelle la teneur en eau est de 30 à 90 % en masse, de préférence de 30 à 80 % en masse ; et
dans laquelle les agents fournissant une apparence floutée sont choisis parmi une poudre de Nylon-6 ; du poly(méthacrylate de méthyle) ; une microsphère de polyuréthane ; un copolymère d'éthylène-méthacrylate de méthyle ; du mica et du dioxyde de titane et un copolymère d'éthylène/méthacrylate et du triisostéarate d'isopropyltitane ; du Nylon-12 et du chitosane ; et de la silice et du phosphate de fluoroalcool en C9-15.

**2.** Composition selon la revendication 1, dans laquelle les particules hydrophobiquement modifiées comprennent de la silice ou de l'oxyde de titane, comprennent encore mieux de la silice.

**3.** Composition selon la revendication 1 ou la revendication 2, dans laquelle la silice hydrophobiquement modifiée comprend

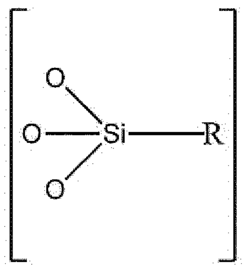

où R est un groupe alkyle en C4 à C18, encore mieux un groupe alkyle en C8H17.

**4.** Composition selon l'une quelconque des revendications 1 à 3, dans laquelle la particule hydrophobiquement modifiée présente une taille de particule primaire de 1 nm à 100 nm, de préférence de 5 nm à 70 nm.

**5.** Composition selon l'une quelconque des revendications 1 à 4, dans laquelle le silicone amino fonctionnalisé comprend un copolymère d'aminoglycol.

**6.** Composition selon l'une quelconque des revendications 1 à 5, dans laquelle la teneur en silicone amino fonctionnalisé est de 0,05 à 25 % en masse, de préférence de 0,05 à 15 % en masse, encore mieux de 0,1 à 10 % en masse et bien mieux encore de 0,2 à 5 % en masse de la composition totale.

**7.** Composition selon l'une quelconque des revendications 1 à 6, dans laquelle le rapport massique de particule hydrophobiquement modifiée à silicone amino fonctionnalisé est de 3:1 à 1:50, de préférence de 1:1 à 1:10.

**8.** Composition selon l'une quelconque des revendications 1 à 7, dans laquelle l'agent de modification de la viscosité comprend au moins un silicone non-amino fonctionnalisé et l'agent de modification de la viscosité est de préférence choisi dans le groupe constitué de polydiméthylsiloxane, pentasiloxane, dimethicone, huile minérale et mélanges de ceux-ci.

**9.** Composition selon l'une quelconque des revendications 1 à 8, dans laquelle la teneur d'agent de modification de la viscosité est de 0,05 à 60 % en masse, de préférence de 1 à 40 % en masse et encore mieux de 5 à 30 % en masse.

**10.** Composition selon l'une quelconque des revendications 1 à 9, dans laquelle le rapport massique d'agent de modification de la viscosité à silicone amino fonctionnalisé est de 10:1 à 1:50 et de préférence de 3:1 à 1:20.

**11.** Composition selon l'une quelconque des revendications 1 à 10, dans laquelle l'agent de modification de lumière optique comprend du dioxyde de titane.

**12.** Composition selon l'une quelconque des revendications 1 à 11, dans laquelle la teneur en agent de modification de lumière optique est de 0,01 à 20 % en masse, de préférence de 0,1 à 10 % en masse et encore mieux de 0,1 à 5 % en masse.

**13.** Composition selon l'une quelconque des revendications 1 à 12, dans laquelle la viscosité de la composition à 25 degrés Celsius est de $10^3$ Pa.s à $10^5$ Pa.s, et de préférence de $5*10^4$ Pa.s à $5*10^5$ Pa.s.

**14.** Composition selon l'une quelconque des revendications 1 à 13 présentant un paramètre de durabilité d'apparence optique OAD après 30 minutes d'au moins 50 %, de préférence d'au moins 60 %, encore mieux d'au moins 70 %, bien mieux encore d'au moins 80 %, et particulièrement de préférence d'au moins 90 % ; dans laquelle le OAD est défini comme l'opacité ou le flouté après un temps déterminé (t) divisé par l'opacité ou le flouté directement après l'application (t=0) multiplié par 100 % ; dans laquelle l'opacité est mesurée par un spectrophotomètre portable (MINOLTA CM2600d en mode SCE) à une température d'environ 23 degrés Celsius et une humidité relative de 45 % avec un film produit avec une épaisseur de 75 micromètres appliqué sur une carte noire et blanche en utilisant un dispositif d'application de film, avec l'opacité (450 nm) étant le rapport entre le coefficient de réflexion du film produit sur les cartes noires et blanches à une longueur d'onde de 450 nm ; et dans laquelle l'indice de flouté est mesuré avec un goniomètre après application d'un film produit de 75 micromètres sur la lame de verre en utilisant un dispositif d'application de film cubique SHEEN à une température d'environ 23 degrés Celsius et une humidité relative de 45 %, avec le flouté (SF) du film produit étant calculé avec :

$$SF = \int_{ST+9}^{ST+90} T(\theta)d\theta / \int_{ST}^{ST+90} T(\theta)d\theta$$

où $T(\theta)$ est le facteur de transmission à angle $\theta$ ; ST est le facteur de transmission spéculaire ; et l'angle incident est fixé à 48°.

**15.** Procédé de modulation de l'apparence optique de la peau comprenant l'étape d'application à la peau d'une composition cosmétique comprenant une émulsion eau-dans-huile selon l'une quelconque des revendications â 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2431103 A
- JP 2008143820 A
- EP 2280040 A
- US 2008152681 A
- US 6268454 B
- US 7282236 B
- US 4185087 A
- WO 9631188 A


### Non-patent literature cited in the description


- **DR. EMMERT.** Quantification of the Soft-Focus Effect. *Cosmetics and Toiletries,* 1996, vol. 111, 57-61
- **BAUER, K. et al.** Common Fragrance and Flavor Materials. VCH Publishers, 1990
- **S. GU et al.** *Journal of Colloid and Interface Science,* 2005, vol. 289, 419-426